# EUROPEAN PATENT APPLICATION

(11) **EP 4 670 688 A2**
(43) Date of publication of application: **31.12.2025**
(21) Application number: 25202304.9
(22) Date of filing: 25.03.2022
(51) Int. Cl.: A61F 7/00

(54) **COMBINED TREATMENT OF BRAIN INJURY**

(30) Priority: 25.03.2021 EP 21165013
(62) Divisional of application: 22713959.9
(71) Applicant: Chiesi Farmaceutici S.p.A., 43122 Parma (IT)
(72) Inventor: IMBIMBO, Bruno Pietro, 43122 Parma (IT); Facchinetti, Fabrizio, 43122 Parma (IT); VILLETTI, Gino, 43122 Parma (IT); Landucci, Elisa, 43122 Parma (IT); Mango, Dalila, 43122 Parma (IT)
(74) Representative: Chiesi Farmaceutici S.p.A.

(57) **Abstract**

The present invention relates to a combined therapy for use in treatment and prevention of treatment and prevention of brain injury, in particular ischemic and/or hypoxic brain lesions, including but not limited to hypoxic-ischemic encephalopathy, preferably in a neonatal subject either at term or at preterm.

## Description

### FIELD OF THE INVENTION

The present invention relates to a combined therapy for use in treatment and prevention of treatment and prevention of brain injury, in particular ischemic and/or hypoxic brain lesions, including but not limited to hypoxic-ischemic encephalopathy, preferably in a neonatal subject either at term or at preterm.

### BACKGROUND OF THE INVENTION

Nerve growth factor (NGF or hNGF) is a neuroactive protein whose discovery was rewarded with the Nobel Prize for Medicine (1986, Rita Levi-Montalcini). Over the decades, its therapeutic potential has been extensively studied, in particular in chronic/degenerative diseases. An ocular formulation of recombinant human NGF (rhNGF) has recently been approved by EMA and FDA for the treatment of neurotrophic keratitis, a rare ocular disease. Less investigated is the neuroprotective potential of hNGF in acute lesions of the central nervous system (CNS). Some scientific publications have indicated that a deficit in the production of endogenous neurotrophic factors may be a contributing factor in determining the severity of the long-term neurological deficits observed after neonatal hypoxic-ischemic damage (Wang et al., Int J Neurosci 2013; 123: 191-195), thus suggesting their administration in the acute phase of the lesion (Acta Cir Bras 2017; 32: 270-279, Int J Clin Exp Med 2013; 6: 951-955). In particular, NGF administration limits the deficit in the development and maturation of complex behaviors assessed at a young age. Unfortunately, hNGF also has a powerful hyperalgesic effect (Nat Med 1995; 1: 774-780). It is known that (wild-type) NGF affects target cells through binding to two separate receptors, (a) receptor tyrosine kinase A (TrkA), which promotes neuronal survival, and (b) p75 neurotrophin receptor, which is involved in the regulation of cell death (Mesentier-Louro et al., 2017, Int. J. Mol. Sci., vol. 18(98), so that polypeptide sequence elements with wild-type NGF, following optic nerve crush, may also have the effect of exacerbation of retinal degeneration via stimulation of apoptosis, see Mesentier-Louro et al., 2018, Mol. Neurobiol., vol. 56, p. 1056-1069. It is also known that the residue R100 of wild-type human NGF is involved in binding of NGF to p75, and that mutation of that residue affects p75 binding, see e.g. WO 2008/006893 A1. In contrast to wild-type NGF, hNGF, the polypeptide of the present invention has a lower binding affinity for p75 (see e.g. Malerba et al., 2015, PlosOne, 10(9): e0136425). Human NGF P61 SR100E corresponds to the polypeptide of SEQ ID NO: 1, also named CHF6467 (SEQ ID No. 1).

Hypoxic-ischemic encephalopathy (HIE) or neonatal asphyxia is a major cause of severe neurological disability and perinatal mortality. In Western Europe and in the US, its incidence is estimated to be 1.5-2 cases out of 1,000 live births, with a higher prevalence in premature infants (Pediatr Res 2013; 74, Suppl 1, 50-72). Neuronal death following HIE occurs by apoptosis or autophagy mediated by programmed cell death mechanisms, or most likely by an apoptosis-necrosis cell death continuum (Ann Neurol 2011; 69: 743-758), triggered by a cascade of events which include the release of excitotoxic glutamate, activation of NMDA and AMPA receptors, intracellular Ca2+ overload, mitochondrial impairment, oxidative stress, caspase activation and inflammation (Lancet Neurol 2011; 10: 372-382).

Undoubtedly, brain damage following HI insult is a complex process with multiple contributing mechanisms and pathways resulting in both early and delayed injury (Patel, S. et al. (2014). Biochem. Soc. Trans. 42, 564-568). Thus, novel treatments to be added to therapeutic hypothermia, the current standard of care (Davidson, J. O., et al., (2015). Front. Neurol. 6.), are urgently needed to increase neuroprotection. Moreover, there is evidence that therapeutic hypothermia is more effective against moderate than against severe hypoxic-ischemic encephalopathy (Sabir, H., et al., (2012). Stroke 43, 3364-3370). It is increasingly recognized that one of the leading pathogenic factors of neonatal brain damage is inflammation, induced by activation of the central and peripheral immune system (Hagberg et al., (2015). Nat. Rev. Neurol. 11, 192-208). Immune responses are induced within minutes and can persist for weeks and even months after the insult (Fleiss et al., 2015 Dev. Med. Child Neurol. 57, 17-28). Hypothermia, the gold standard intervention after a hypoxic-ischemic insult, is not beneficial in all treated newborns (Bainbridge et al., 2012. Brain 136, 90-105). Nerve growth factor (NGF) has been shown to protect specific neurons that express its signaling receptor, trkA, from a variety of insults (Holtzman et al., 1995 J. Neurosci. 15). There are some data, in particular in the developing brain, indicating that NGF has neuroprotective actions that extend beyond neuronal cells (Fodelianaki et al., 2019 Exp. Cell Res. 377). Indeed, NGF is neuroprotective in animal models of newborn hypoxic-ischemic encephalopathy and it has long been suggested as a promising agent for protecting neonatal brain from hypoxic-ischemic insults (Holtzman et al., 1996, Ann. Neurol. 39. doi:10.1002/ana.410390117).

Therapeutic hypothermia is the standard of care for improving survival and reducing the neurodevelopmental sequelae in neonates with moderate to severe hypoxic-ischemic encephalopathy (HIE). Selective head cooling or whole-body cooling to a target core temperature of 33.5°C for around 72 h commencing within a therapeutic window of 6 h after birth improves survival and the neurological outcome in neonates with HIE (J Pediatr 2015; 91: S78-S83). Although relatively few preclinical studies have compared multiple hypothermic treatment temperatures, it has been recently proposed that, since cooling below 33.5°C does not appear to provide additional neuroprotection in neonatal rat models of HIE, relatively mild cooling should be considered for future clinical trials (Sci Rep 2016; 6: 23430). Hypothermia, although not being associated with major complications, appears to be beneficial only to a relatively small percentage of infants with HIE (Int J Mol. Sci 2015; 16: 22368-22401). Specifically, treatment with therapeutic hypothermia is currently reserved for newborns born at term or near term (> 1800 g) and is usually restricted to Reference Centers with specific expertise and equipment.

WO 2018087656 relates to pharmaceutical compositions comprising NGF or molecules with NGF-like activity for use in the treatment of traumatic of brain injury, in particular traumatic brain lesion or brain ischemic hypoxic lesion, including but not limited to hypoxic-ischemic encephalopathy by means of intranasal administration.

Hou et al. (Chinese Journal of Primary Medicine and Pharmacy 2017;24(3):338-341) report that head hypothermia combined with mouse nerve growth factor therapy for the treatment of neonatal moderately severe HIE has protective effect. As mentioned above, the use of hypothermia in the treatment of neonatal asphyxia has been well documented in the art (see for example, Volpe, 2001; Gunn et al, 2000). However, to date there has been no teaching or suggestion in the art that hypothermia could be use in combination with the administration of mutant NGF, CHF6467. Nor has there been any suggestion that such combination therapy would lead to such a surprising and unexpected enhancement in the resulting neuroprotective effect.

Therefore, there is still the need for agents for the treatment of HIE. In particular, agents capable of enhancing the effects of hypothermia may be an extremely valuable addition to current clinical practice.

### SUMMARY OF THE INVENTION

CHF6467 (hNGFp, SEQ ID No. 1) has similar neurotrophic activity as wild type human nerve growth factor (NGF), but its pain sensitizing activity is tenfold lower (Cattaneo, A., and Capsoni, S. (2019). Pharmacol. Res. 139. doi: 10.1 016/j .phrs.2018.1 0.028.).

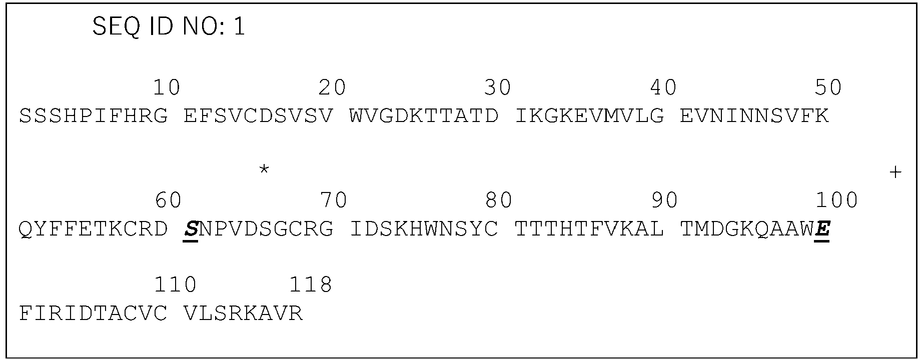

The invention provides a combination of a polypeptide of SEQ ID NO: 1 and hypothermia for use in treatment and/or prevention of traumatic brain lesion or of brain ischemic hypoxic lesion in a mammalian subject.

In a preferred embodiment the the polypeptide is administered at a dose of 20 µg/kg.

In a preferred embodiment the traumatic brain lesion is hypoxic-ischemic encephalopathy.

In a preferred embodiment the mammalian subject is a human, preferably the subject is a neonatal or newborn subject or a premature newborn subject, preferably the neonatal or newborn subject is affected by moderate to severe hypoxic-ischemic encephalopathy

In a preferred embodiment the polypeptide is administered intranasally, or wherein the polypeptide is administered repeatedly, preferably for a period of 1 to 30 days, preferably 2 to 14 days. Repeated cycles of treatment are also envisaged.

In a preferred embodiment the polypeptide is administered repeatedly between 1 and 7 times per day, or the polypeptide is administered to one or both nostrils.

In a preferred embodiment the polypeptide is administered at a dose of 0.01 to 1.00 mg per day, preferably at a dose 0.06 to 0.12 mg/day of the polypeptide, preferably at a dose of 0.06 mg/day.

Preferably the amount of 0.06 mg/day is for a neonate or new born of about 3 kg of body weight.

Prefered doses, in particular for subject with higher body weight, are 3 to 333 µg/kg.

Prefered doses are of about 20 µg/kg to about 40 µg/kg, preferably 20 µg/kg.

In a preferred embodiment when the polypeptide is administered repeatedly, each dose is of 0.01 to 1.00 mg per day, preferably each dose is of 0.06 to 0.12 mg/day of the polypeptide, preferably each dose is of 0.06 mg/day.

In a preferred embodiment the polypeptide is administered within about 1 hour to 24 hours after the brain lesion.

In a preferred embodiment the polypeptide is comprised in a composition comprising an acetate buffer and/or methionine.

In a preferred embodiment the polypeptide is administered simultaneously with hypothermia and/or no more than 24 hours after hypothermia.

In a preferred embodiment the hypothermia is to be inititated within 1 hour to 10 hours after the hypoxic-ischemic (HI) insult, preferably the hypothermia is to be inititated within 2 hours to 8 hours after the hypoxic-ischemic (HI) insult, preferably the hypothermia is to be inititated within 4 hours to 6 hours after the hypoxic-ischemic (HI) insult.

In a preferred embodiment the hypothermia is to be maintained for a period of from about 1 to about 72 hours after the hypoxic-ischemic (HI) insult, preferably of at least about 4 hours, more preferably of at least about 6 hours, more preferably of at least about 12 hours after the hypoxic-ischemic (HI) insult.

Preferably, the hypothermia is maintained for a period of at least about 4 hours, more preferably at least about 6 hours or 12 hours, after birth.

In a preferred embodiment the temperature of the mammal is to be maintained at a temperature of from about 32°C to about 36°C, preferably from about 32°C to about 35°C.

Another aspect of the invention relates to treating neonatal asphyxia in a mammal in need thereof by
(a) administering a therapeutically effective amount of CHF6467 to the mother of the mammal prior to and/or during labour; and
(b) subjecting the mammal to hypothermia after birth.

Hypothermia may be produced passively, by allowing the temperature to drift downwards and not purposefully sustain body temperature. Being poikilothermic, neonates rapidly assume the temperature of their surroundings. Alternatively the patient may be actively rendered hypothermic by deliberately reducing their ambient temperature.

A further aspect of the invention relates to treating neonatal asphyxia in a mammal in need thereof by:
(a) administering a therapeutically effective amount
   of CHF6467 to the mammal; and
(b) subjecting the mammal to hypothermia, or hypothermic conditions.

In a preferred embodiment, the mammal is a newborn subject in the first four weeks after birth. More preferably, the mammal is in the first two weeks, more preferably still, the first week after birth.

Preferably, the mammal is a human.

Preferred embodiments for the second aspect of the invention are the same as those described above in respect of the first aspect.

Another aspect of the invention relates to treating neonatal asphyxia in a mammal in need thereof by administering a therapeutically effective amount of xenon
to the mammal in combination with hypothermia.

Yet another aspect of the invention relates to the use of CHF6467 in the preparation of a medicament for the treatment of neonatal asphyxia, wherein said treatment comprises administering to a subject simultaneously, sequentially or separately CHF6467 in combination with hypothermia.

A further aspect of the invention relates to the use of CHF6467, in combination with hypothermia, for the treatment of neonatal asphyxia.

In a preferred embodiment in the combination for use in treatment and/or prevention of traumatic brain lesion or of brain ischemic hypoxic lesion in a mammalian subject, the polypeptide is administered at a dose 0.06 to 0.12 mg/day and the temperature of the mammal is to be maintained at a temperature of from about 32°C to about 35°C.

In a preferred embodiment in the combination for use in treatment and/or prevention of traumatic brain lesion or of brain ischemic hypoxic lesion in a mammalian subject, the polypeptide is administered at a dose of about 20 µg/kg to 40 µg/kg and the temperature of the mammal is to be maintained at a temperature of from about 32°C to about 35°C.

The following figures and non-limiting examples illustrate the invention without limiting its scope.

### DETAILED DESCRIPTION OF THE DRAWINGS

**Figure 1****.** Effects of CHF6467 on baseline transmission at CA1 hippocampal synapses. **Figure 2****.** CHF6467 reverted depression induced by OGD (preventive setting). Normalized pooled data show the effects of OGD on field excitatory postsynaptic potentials (fEPSPs) and the protective effects of CHF6467 perfused at different concentrations (0.03 and 0.1 µg/mL) during OGD.
**Figure 3****.** EPSC depression induced by OGD is restored by CHF6467 perfusion (therapeutic setting). Panel A shows the time course of normalized EPSC data after OGD alone (closed cricles) and after OGD + CHF6467 (open circles). Histogram shows the average normalized EPSC data in the last 10 minutes of receording after OGD and OGD + CHF6467. Panel B shows that CHF6467 does not induce any change on baseline transmission in the absence of OGD.
**Figure 4****.** Synergic effect of CHF6467 with hypothermia against OGD. Normalized pooled data showing the synergic effect of hypothermia and CHF6467 perfused following a 15-min period of OGD.
**Figure 5****.** Diagram of the experimental protocol of the rat model of neonatal hypoxic-ischemic encephalopathy. TTC = 2,3,5-Triphenyltetrazolium chloride
**Figure 6****.** Effects of intranasal CHF6467 (20 and 40 µg/kg) alone and in combination with hypothermia on Log-transformed brain infarct volume in juvenile rats 72h after the hypoxic-ischemic insult at 10 days of age. Numbers of animals are indicated above each column.
**Figure 7****.** Hippocampal mRNA levels of a panel of rat cytokines and chemokines in sham-operated, vehicle-treated hypoxic-ischemic encephalopathy (HIE), HIE treated with CHF6467, 20 ug/kg (HIE+CHF 20ug/ml), HIE treated with hypothermia (HIE+IPO), HIE treated with CHF6467 and hypothermia (HIE+IPO+CHF). Scatter plots with means ± standard error mean (SEM). ** p <0.01 and * p<0.05 vs HIE.
**Figure 8****.** Cortical mRNA levels of a panel of rat cytokines and chemokines in sham-operated, hypoxic-ischemic encephalopathy (HIE), HIE treated with CHF6467, 20 ug/kg (HIE+CHF 20ug/ml), HIE treated with hypothermia (HIE+IPO), HIE treated with CHF6467 and hypothermia (HIE+IPO+CHF). Scatter plots with means ± standard error mean (SEM). ** p<0.01 and * p<0.05 vs HIE.
**Figure 9****.** Statistical analysis of mean log10-transformed brain infarcted volume by treatment group.
**Figure 10****.** CHF6467 brain penetration after intranasal administration in control juvenile rats as determined by the quantification of SSSHPIFHR (SEQ ID No. 2) peptide.
**Figure 11****.** CHF6467 brain penetration after intranasal administration in juvenile rats subjected to hypoxic-ischemic insult. Light to heavy ratios of CHF6467-specific peptides SSSHPIFHR (SEQ ID No. 2) (A) and QAAWEFIR (SEQ ID No. 3) (B) and unspecific peptide QYFFETK (SEQ ID No. 4) (C) in different rat brain areas.

### DETAILED DESCRIPTION OF THE INVENTION

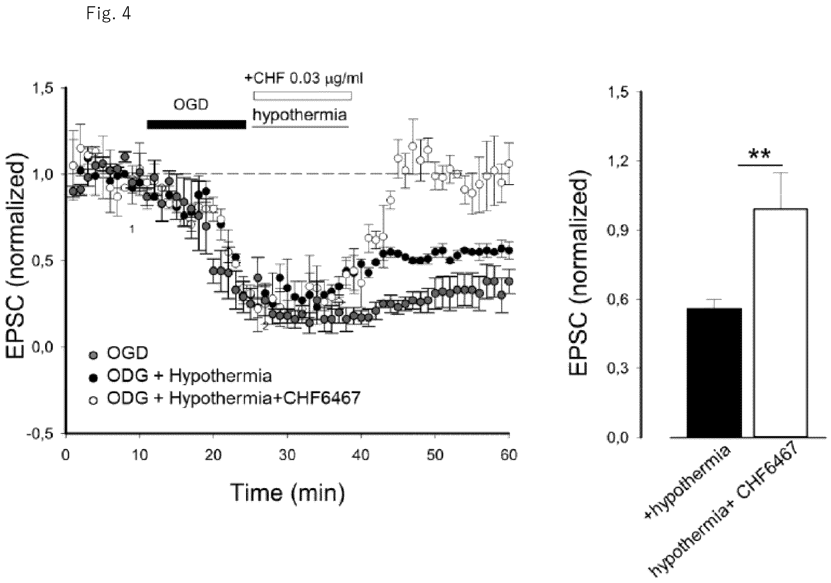

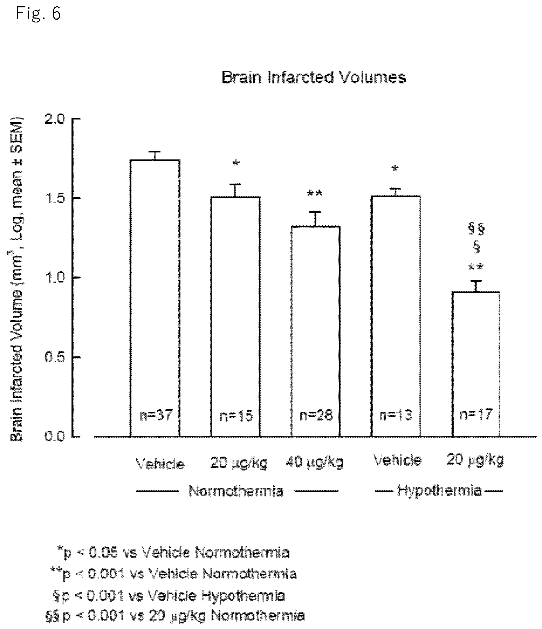

The neuroprotective effects of CH6467 or polypeptide of SEQ ID No. 1 were studied using in vitro and in vivo in experimental models of hypoxic-ischemic encephalopathy (HIE). In acute hippocampal slices undergoing oxygen-glucose deprivation (OGD), an established in vitro model of hypoxic-ischemic brain injury, CHF6467 significantly attenuated abnormal excitatory post synaptic currents (EPSC). Unexpectedly, the combination of CHF6467 and hypothermia completely reversed abnormal EPSC (Fig. 4). In neonatal rats subjected to permanent left common carotid artery occlusion followed by hypoxia, an established model of neonatal hypoxic-ischemic encephalopathy, intranasal administration of CHF6467 (20 and 40 µg/kg) significantly decreased the volume of infarcted brain compared to vehicle-treated animals. Surprisingly, the combination of CHF6467 and hypothermia dramatically reduced the volume of infarcted brain by 86% compared to vehicle-treated animals in normothermia. Unexpectedly, the neuroprotective effects of the combination of CHF6467 and hypothermia were much greater than those of the single agents (CHF6467 or hypothermia) alone indicating a synergic effect (Fig. 6). In particular, the effects of a preferred dose (20 µg/kg) of CHF6467 *in vivo* synergized with hypothermia in affording neuroprotection. No mortality, clinical signs or weight loss were observed upon CHF6467 treatments suggesting that CHF6467 appears well tolerated.

Then the present invention provides the combined use of CHF6467 and hypothermia in the treatment of brain injury, traumatic brain lesion or brain ischemic hypoxic lesion, preferably hypoxic-ischemic encephalopathy. In particular the combination of CHF6467 and hypothermia may significant decrease mortality and severe neurodevelopmental disabilities compared to hypothermia alone in infants and neonates with moderate-to-severe hypoxic-ischemic encephalopathy, particularly when proper dosages are considered, as reported in the herein below experimental part.

Further, the modulatory effects of CHF6467, alone or in combination with hypothermia, against a panel of neuroinflammatory markers in the cortex and hippocampus of rat pups subjected to hypoxic-ischemic brain injury were also investigated.

A marked increase of mRNA levels of several cytokines and chemokines in the hippocampus and the frontoparietal cortex was measured in rats that underwent hypoxic-ischemic insult in comparison with sham-operated rats. CHF6467 (20 ug/kg, intranasal) significantly counteracted the upregulation of several neuroinflammatory markers in the hippocampal region of rats subjected to the hypoxic-ischemic insult while hypothermia per se was not as effective as CHF6467. In the cortex, CHF6467 (20 ug/kg) per se was effective in counteracting some neuroinflammatory markers upregulation induced by the hypoxic-ischemic insult and such effect was in some cases augmented by the combination with hypothermia.

Each of the references cited herein (including all patents, patent applications, scientific publications, manufacturer's specifications, instructions, presentations, etc.), whether above or below, are hereby incorporated by reference in their entirety. Nothing herein is to be construed as an admission that the present invention would not be entitled to antedate a specific teaching and/or as an admission that a specific reference, other than the common general knowledge, contains information sufficiently clear and complete for it to be carried out by a person skilled in the art.

The expression "and/or", e.g., "X and/or Y" shall be understood to mean either "X and Y" or "X or Y" and shall be taken to provide explicit disclosure of "and", of "or" and of both meanings ("and" or "or").

As used herein, unless specified otherwise, the terms "about", "ca." and "substantially" all mean approximately or nearly, and in the context of a numerical value or range set forth herein preferably designates +/- 10 %, more preferably +/- 5 %, around the numerical value or range recited or claimed.

Unless expressly specified otherwise, the word "comprise", or variations such as "comprises" or "comprising" is used in the context of the present document to indicate that further members may optionally be present in addition to the members of the list introduced by "comprising". It is, however, contemplated as a specific embodiment of the present invention that the term "comprising" encompasses the possibility of no further members being present, i.e. for the purpose of this embodiment "comprising" is to be understood as having the meaning of "consisting of".

Unless expressly specified otherwise, all indications of relative amounts regarding the present invention are made on a weight/weight basis. Indications of relative amounts of a component characterized by a generic term are meant to refer to the total amount of all specific variants or members covered by said generic term. If a certain component defined by a generic term is specified to be present in a certain relative amount, and if this component is further characterized to be a specific variant or member covered by the generic term, it is meant that no other variants or members covered by the generic term are additionally present such that the total relative amount of components covered by the generic term exceeds the specified relative amount; more preferably no other variants or members covered by the generic term are present at all.

Unless the context dictates otherwise, the term nerve growth factor stand for wild-type NGF only and does not include the polypeptide of SEQ ID NO: 1.

The terms "NGF mutein" and "mutein of NGF", or, with reference to NGF "mutein thereof", are used herein interchangeably to refer to a polypeptide which is characterized by at least one mutation, compared to wild-type NGF, as further described in detail herein. The polypeptide of SEQ ID NO: 1 is a mutein of NGF. It refers to any species, preferably mammalian species; however, human NGF mutein is always preferred Preferably a mutein of NGF has 80 to 99.5 % sequence identity with NGF, particularly human NGF, more preferably a mutein has 90 to 99% sequence identity with NGF, particularly human NGF.

As used herein, the term "hypothermia" refers to subjecting a particular subject (for example a neonatal subject) to hypothermic conditions, for example, by lowering the body temperature, preferably by 3-5°C, through passive or active techniques. Prefered hypothermia temperature is between 32°C and 36°C, preferably 32°C to 35°C, preferably 32°C or 33°C.

As used herein, "simultaneously" is used to mean that CHF6467 is administered concurrently with hypothermia, whereas the term "in combination" is used to mean the CHF6467 is administered, if not simultaneously, then "sequentially" within a timeframe in which CHF6467 and the hypothermia both exhibit a therapeutic effect, i.e. they are both available to act therapeutically within the same time-frame. Thus, administration "sequentially" may permit CHF6467 to be administered within 5 minutes, 10 minutes or a matter of hours before or after the hypothermia, provided the circulatory half-life or target tissue residence time of CHF6467 is such that it is present in a therapeutically effective amount when the neonatal subject is exposed to hypothermic conditions.

The terms "patient" and "subject" are used interchangeably herein, particularly with reference to a patient/subject characterized by a ophthalmic disorder, as described herein.

In the context of the present invention, the term "prevent" is to be understood broadly and includes not only the prevention of onset of the brain injury, but also the prevention of progression of the brain injury. In particular, in the context of a disorder involving a damage and/or disorder of the brain, the term "prevent also includes the prevention of further progression of the damage of the brain.

In the context of the present invention, the term "treat" is to be understood broadly and includes without limitation the amelioration of the symptoms of the disorder.

According to the present invention, an "effective amount" is the amount or dose which achieves a desired reaction or a desired effect, either alone or together with further doses.

The term "pharmaceutically acceptable" generally describes that a certain substance can be administered to a subject, optionally and preferably in combination with an agent, without the agent causing intolerable adverse effects, at the dosage used.
The terms "pharmaceutically acceptable carrier" and "pharmaceutically acceptable excipient" are used to refer to any one or more of solvents, dispersion media, coatings, antibacterial and antifungal agents, isotonic and absorption delaying agents, and the like that are physiologically compatible and are suitable for administration to a subject as described herein, or do not otherwise interfere with such administration. Examples of such pharmaceutically acceptable carriers comprise without limitation one or more of water, saline, phosphate buffered saline, dextrose, glycerol, ethanol, polyorbate 80 and the like, as well as combinations thereof. Particularly for the case of liquid pharmaceutical compositions, it may be preferable to include isotonic agents, for example, sugars, polyalcohols such as mannitol, sorbitol, or sodium chloride in the composition.

Pharmaceutically acceptable carriers may further comprise auxiliary substances such as wetting or emulsifying agents, preservatives or buffers, which enhance the shelf life or effectiveness of the agent. A pharmaceutically acceptable carrier is typically comprised in a composition according to the present invention.

The term "pharmaceutically active agent" refers to an agent that can be used in the administration to a subject where the agent would be of benefit, e.g., in ameliorating the symptoms of a disease or disorder. In addition, a "pharmaceutically active agent" can have a positive or advantageous effect on the condition or disease state of a subject when administered to the subject in a therapeutically effective amount. Preferably, a pharmaceutically active agent has curative properties and may be administered to ameliorate, relieve, alleviate, reverse, delay onset of or lessen the severity of one or more symptoms of a disease or disorder. A pharmaceutically active agent may have prophylactic properties and may be used to delay the onset of a disease or to lessen the severity of such disease or pathological condition. For example, an agent of the invention is considered herein as a pharmaceutically active ingredient for the treatment of cystic fibrosis, as claimed. In another example, a pharmaceutically active protein can be used to treat a cell or an individual which does not normally express a protein, or not at the desired levels, or which mis-expresses a protein, e.g., a pharmaceutically active protein can compensate for a mutation, or for lack of sufficiently high expression, by supplying a desirable protein. The term "pharmaceutically active peptide or protein" includes entire proteins or polypeptides, and can also refer to pharmaceutically active fragments thereof. It can also include pharmaceutically active analogs of a peptide or protein.

The terms "subject" and "patient", as used herein, relate to a mammal. For example, mammals in the context of the present invention are humans, non-human primates, domesticated animals including but not limited to dogs, cats, sheep, cattle, goats, pigs, horses etc., laboratory animals including but not limited to mice, rats, rabbits, etc., as well as animals in captivity such as animals of zoos. The terms "subject" and "patient" as used herein particularly include humans. The subject (human or animal) has two sets of chromosomes; that is, the subject is diploid. The term "patient" refers to a subject which suffers from a condition, is at risk of suffering from a condition, has suffered from a condition, or is predicted to suffer from a condition, and which may be subjected to therapy, e.g. by administration of an agent. The patient's condition may be chronic and/or acute. Thus, a "patient" can also be described as a subject subjected to a therapy and/or or in need of a therapy.

The term "therapy" is to be understood broadly and refers to the treatment of a subject with the goal to prevent or treat a condition in the subject. In preferred embodiments, therapy specifically includes the administration of an agent to the subject.

The agent according to the present invention, also termed herein "polypeptide of SEQ ID NO: 1" will now be described in more detail. The term "polypeptide of SEQ ID NO: 1" and similar terms denote herein a polypeptide comprising the amino acid sequence defined by SEQ ID NO: 1 and/or an agent with equivalent biological activity. Thus, within these terms are also included functionally equivalent parts or analogues of such polypeptides. One example of a biologically equivalent part of the polypeptide could be a domain or subsequence of the polypeptide of SEQ ID NO: 1, which includes the binding site to enable the domain or subsequence to exert substantially the same biological activity as the full-length polypeptide of SEQ ID NO: 1 or alternatively a gene coding for such a polypeptide. The term "substantially the same biological activity" refers to an equivalent part or analogues polypeptide having at least 50%, preferably at least 60%, more preferably at least 70%, more preferably at least 75%, more preferably at least 80%, more preferably at least 85%, more preferably at least 90%, more preferably at least 95% and most preferably at least 97%, at least 98% or at least 99% of the activity of the polypeptide of SEQ ID NO: 1 in the assays described in Examples 1-3. An example of a biologically equivalent analogue of the polypeptide could be a fusion protein which includes at least a part of the amino acid sequence of the polypeptide of SEQ ID NO: 1, but it can also be a homologous analogue of the polypeptide. Also, completely synthetic molecules that mimic the specific biological activity of the polypeptide of SEQ ID NO: 1 would constitute "biologically equivalent analogues".

More preferably, the term "polypeptide of SEQ ID NO: 1" and similar terms denote herein a polypeptide comprising the amino acid sequence defined by SEQ ID NO: 1; such agents are optionally fusion proteins which comprise inter alia the amino acid sequence defined by SEQ ID NO: 1. Most preferably, the term "polypeptide of SEQ ID NO: 1" and similar terms denote herein a polypeptide consisting of the amino acid sequence defined by SEQ ID NO: 1; in this embodiment, the agent consists of a polypeptide consisting of the 118 amino acid residues in sequential order as defined by SEQ ID NO: 1. In this and other embodiments, the polypeptide optionally carries one or two or three internal cysteine bonds, so that cysteine (Cys, C) residues are covalently linked to each other to form intramolecular disulfide bridges. The cysteine bonds are preferably equivalent to those in wild-type human NGF.

The polypeptide of the present invention may optionally be characterized by further post-translational modifications. Such post-translational modifications optionally include glycosylation and/or phosphorylation. Preferably, however, the polypeptide according to the present invention is free of glycosylation and/or phosphorylation. Indeed, considering that the experimental examples herein demonstrate a beneficial effect on the healing of of brain injury, in particular traumatic brain lesion or brain ischemic hypoxic lesion, including but not limited to hypoxic-ischemic encephalopathys and a beneficial benefit-to-adverse effect ratio, whereby the polypeptide used was obtained by cytosolic recombinant expression in bacteria, which typically does not result in glycosylation and/or phosphorylation, it is plausible that the beneficial effect of the present invention is not dependent on such type of post-translational modification.

Typically, the polypeptide according to the present invention is a non-natural polypeptide which is not naturally produced by the subject to which the polypeptide is administered.

Preferably the polypeptide according to the present invention is an isolated polypeptide. More preferably, the polypeptide according to the present invention is essentially free of host cell proteins, degradation products (such as des-nona variant, for example), and protease (such as trypsin, for example). When the polypeptide according to the present invention is essentially free of host cell proteins, degradation products (such as des-nona variant, for example), and protease (such as trypsin, for example) is may also be referred to as "pure polypeptide". Preferably, the polypeptide according to the present invention is administered as pure polypeptide. More preferably, the pure polypeptide consisting of SEQ ID NO: 1 1 has a weight percentage of 90 % or more, preferably 92 % or more, more preferably 93 % or more, more preferably 94 % or more, more preferably 96 % or more, more preferably 97 % or more, more preferably 98 % or more, more preferably 99 % or more, more preferably 99.2 % or more, more preferably 99.4 % or more, more preferably 99.6 % or more, more preferably 99.8 % or more, more preferably 99.9 % or more, with respect to the total protein in the composition. Most preferably, the pure polypeptide according to the present invention has a purity grade compatible with Good manufacturing practices (GMP).

The polypeptides consisting of SEQ ID NO: 1, differ in two positions from the amino acid sequence of human nerve growth factor (NGF, also referred to as wild-type human NGF or wild-type NGF). The difference of the polypeptide according to the present invention with respect to the wild-type polypeptide has a remarkable effect on the treatment or prevention of brain injury, in particular traumatic brain lesion or brain ischemic hypoxic lesion, including but not limited to hypoxic-ischemic encephalopathy, and the absence of side effects, as disclosed in detail herein and supported by the experimental examples herein.

Among several mutants of human NGF (hNGF mutants), the mutant human NGF P61SR100E is considered as most promising for brain injury treatment. However, the specific uses of treatment and/or prevention according to the present invention are not instructed by those references, and those references also fail to make the respective polypeptides available at a high enough purity to enable its medical use in humans. Thus, the polypeptide for use according to the present invention, as described and provided herein, provides several unexpected advantages over wild-type human NGF. Administration of the agent according to the present invention is causative of reduction of brain infarcted volume (see e.g. Example 2), and the administration is not associated with pain.

The polypeptide of SEQ ID NO: 1 is not found in nature and can also be referred to as a non-natural polypeptide. Thus, the agent according to the present invention is not wild-type NGF, and in particular not wild-type human NGF.

Preferably, the non-natural polypeptide according to the present invention is provided at high purity. Optionally, the polypeptide is soluble in an aqueous medium.

Preferably, the treatment and/or prevention does not cause side effects or adverse effects in the subject to which the polypeptide is administered or has been administered. Thus, preferably, the administration of the polypeptide of the invention is not causative for any undesired effects in the mammalian subject.

One side effect or adverse effect that is preferably absent in this context is hyperalgesia or pain. It is specifically preferred that the treatment and/or prevention according to the present invention does not cause hyperalgesia in the mammalian subject. Thus, preferably, administration of the agent according to the present invention does not induce any hyperalgesic syndrome (pain).

It is important to point out that the absence of pain does not merely cause a more pleasant (or less unpleasant) treatment than the administration of a reference compound associated with pain (such as wild-type NGF), but is at least in part causative for the success of the treatment or prevention of of brain injury, in particular traumatic brain lesion or brain ischemic hypoxic lesion, including but not limited to hypoxic-ischemic encephalopathy as such: considering that the polypeptide according to the present invention is preferably nasally administered, the absence of pain will enable the treated subject to accept the administration of the polypeptide without adverse reactions such as scraping it off or washing it off or otherwise removing it in order to avoid to pain, and as a result of that, the polypeptide will exert is therapeutically beneficial effect, such as treatment or prevention of brain injury, in particular ischemic brain lesion, including but not limited to hypoxic-ischemic encephalopathy. Thus, the absence of pain associated with the polypeptide of the present invention will be suitable to overcome consumer carer reluctance and concerns of the regulatory authorities. In other words, the absence of pain is associated with a significant increase in the benefit-to-risk ratio compared to agents that are associated with pain.

In particular, preferably, the treatment and/or prevention does not cause hyperalgesia in the mammalian subject. In one embodiment, the subject to which the polypeptide of the invention is administered does not suffer from mechanic allodynia. More precisely, mechanic allodynia is not induced in the subject to which the polypeptide of the invention is administered, so that the subject to which the polypeptide is administered does not suffer from mechanic allodynia.

Typically, administration of the agent according to the present invention is well tolerated by the subject. In particular, preferably, administration of the polypeptide according to the present invention is not associated with the formation of drug-neutralising anti-drug antibodies in the subject. Indeed, as the amino acid sequence of the polypeptide according to the present invention differs in only two amino acid positions from wild type human NGF, it is plausible that the immunological tolerability in humans is particularly advantageous, and it is plausible that administration of the polypeptide of the present invention is not associated with the formation of anti-drug antibodies in humans.

Preferably, administration according to the present invention positively influences one or more of the following: inflammation, innervation and angiogenesis.

The pathologies according to the present invention are generally pathologies characterized by the mechanical and/or pathological destruction (for example degenerative) of the interneuronal connections and death of neurons, glial cells and optionally also vascular tissues at the site affected by such destruction. A non-limiting example of pathologies or conditions according to the present description is constituted by pathologies/conditions deriving from a traumatic event involving the central nervous system, such as brain injuries or traumatic brain injuries (TBIs) or pathologies/conditions associated therewith or directly derived therefrom, such as hypoxic/ischemic brain injuries (HIBIs), or preferably hypoxic-ischemic encephalopathy (HIE) in particular in neonatal patients, infantile cerebral paralysis, or other pathologies/conditions caused by mechanical traumas or by cerebral hypoxia/ischemia.

Injuries such as hypoxic/ischemic encephalopathy (primary or secondary damage), perinatal damage, infantile cerebral paralysis, brain injuries caused by cerebral ischemia of various types, including brain aneurysm ruptures, brain arteriovenous malformations, and brain sinus thrombosis, are also included.

According to the present invention, pathologies caused by changes to the vascular/circulatory system include, for example, hypoxic-ischemic syndromes which include, traumatic injuries; hemorrhagic injuries; atherosclerotic injuries; cardiogenic injuries; hypertensive injuries.

According to the present invention, pathologies caused by inflammatory damage due infective causes such as meningitis/encephalitis, etc, whether of fungal, bacterial, viral, prionic or other etiology.

At pediatric level, such pathologies include, for example, fetal and/or neonatal hypoxia/asphyxiation; major head trauma; acute cerebral hemorrhage, poisoning (carbon monoxide, cyanide); brain sinus thrombosis; coagulation factor deficiency; or also genetic diseases.

According to the present invention, the polypeptide of SEQ ID NO: 1 may be administered to a subject in need of such administration. A subject in need of such administration may be a subject suffering from a disorder described herein, a subject at risk of suffering from such a disorder, or otherwise afflicted with such a disorder. The agent is administered to the subject in a therapeutically effective amount. The therapeutically effective amount can be determined by the physician in view of the disclosure herein.

In particular, the polypeptide according to the invention is administered to a mammalian subject. The subject can also be referred to as "patient". Most preferably, the mammalian subject is a human.

The subject may be a newborn, defined as a baby from the time of birth until 12 weeks of age, while a neonate is defined as a baby from the time of birth until 4 weeks of age. Treatment of newborn and neonate is comtemplate in the present invention. A premature baby is one born before the end of the full term of gestation, while a preterm baby is one born three weeks of more prior to full term.

In the context of the present invention, it is preferred and also demonstrated by the experimental examples herein that preventing brain injury, such as e.g. (partial) reduction of brain infarct or other improvement or amelioration of the condition or disorder, is a preferred integral part of the invention as claimed herein. Indeed, attaining the claimed therapeutic effect is a functional technical feature of the present invention. The examples herein make plausible that said functional technical feature is achievable as a direct result of administration of the polypeptide of the present invention. In other words, the present inventors have identified that the polypeptide of the present invention is causative for achieving amelioration in a subject suffering from a brain injury, in particular in combination with hypotehrmia. The brain injury is preferably characterized by a damage and/or disorder or dysfunction of the brain.

In particular, administration to the brain may be performed by taking advantage of the unique anatomical relationships and functions of the nasal cavity. It has long been recognized that therapeutic materials (including biologics) applied to the area of the cribriform plate in the upper anterior nasal cavity can gain access to the central nervous system via the perforations that admit the olfactory nerves to the nasal cavity from the cranium. Furthermore, for any drug that can be taken up by neural tissues and transported along axonal projections, a supplemental route exists via the olfactory nerves themselves and relevant, nasal branches of the trigeminal nerve, by which therapeutically relevant concentrations of drug may reach many areas of the brain.

In the case of the present invention, intranasal administration will be performed preferably by means of suitable devices for topical administration, which act by means of absorption of the drugs through the nasal mucosa, preferably by the formation of atomized particles with a diameter between 10 and 50 micrometers.

An example of devices suitable for intranasal administration of the composition of the invention is constituted by devices of the MAD (mucosal atomizer device) type or by devices that act with similar mechanisms and are commercially available. The application of the MAD to a syringe enables atomization of the drug as a whole, thus guaranteeing optimal absorption thereof at the nasal mucosa and reducing dispersion to the external environment and to the pulmonary structures to a minimum. Its structure makes it a device that is simple to use and extremely effective.

The administration of polypeptide of the invention intranasally by means of an MAD device is a valid and accessible way of granting this active ingredient access at CNS level.

The device loaded with the composition as described here can thus be used for the administration of polypeptide of the invention at the nasal mucosa and olfactory epithelium, with the objective of carrying it through the olfactory pathways and/or nerve endings, towards the brain parenchyma. The device, combined with the drug, can therefore be used in clinical practice with the objective of inducing neuronal regeneration in the brain, for example for the treatment of the outcomes of brain hypoxic-ischemic injuries or neurodegenerative diseases, treating pathologies or conditions that currently cannot be treated pharmacologically. The compositions described here can be administered in one or more daily doses, with total amounts per day ranging from 0.01 to 1.00 mg per patient.

In general, the polypeptide according to the present invention is administered repeatedly or in a single administration.

In one embodiment, the polypeptide is administered in a single administration. In that embodiment, the polypeptide is administered in a single administration, and administration is discontinued after that single administration.

In an alternative embodiment, the polypeptide is administered repeatedly for a period of one, two or three to 30 days, preferably seven to 14 days. Repeated courses of up to 30 days after a suitable washout period are also envisaged to allow for treatment of chronically progressive or recurrent conditions.

In one embodiment, the polypeptide is administered repeatedly. In one embodiment, the polypeptide is administered repeatedly, e.g. until complete healing of the brain injury, or at least until amelioration of the symptoms of the injury is observed. Alternatively, the polypeptide is administered repeatedly for a period of three to 30 days, preferably seven to 14 days. Optionally, administration is discontinued after completion of said interval. In a particularly preferred embodiment, the polypeptide is administered repeatedly at least three times per day.

Preferably, the polypeptide is administered following a damage of the brain. Alternatively, the polypeptide may be used prophylactically under circumstances of anticipated brain injury or potential brain injury (such as neurosurgical interventions, or developing inflammatory brain conditions).

Preferably, the polypeptide is administered as soon as possible after the diagnosis of brain damage in order to minimize the extent of brain cells death. "as soon as possible" includes embodiments of one day or less after the diagnosis of brain damage or injury. However, the polypeptide is still neuroprotective if administered days after the occurrence of damage of brain damage. Preferably, the polypeptide is administered at least three days after induction of a damage of the brain.

The agents and compositions described herein are administered in effective amounts. In the case of treatment of a particular disorder, the desired reaction preferably relates to inhibition of the course of the disease. This comprises slowing down the progress of the disease and, preferably, interrupting or reversing the progress of the disease. The desired reaction to the treatment of a disease or of a condition may also comprise a delay of the onset or a prevention of the onset of said disease or said condition. In some embodiments the desired reaction comprises the complete healing of the symptoms of the disorder, locally and/or systemically.

An effective amount of an agent or composition described herein will depend on the condition or disorder to be treated, the severity of the disorder, the individual parameters of the subject to which the agent is administered, such as age, physiological condition, accompanying condition(s) (if present), size and weight, the duration of treatment, the type of an accompanying therapy (if present), the specific route of administration and other parameters. Accordingly, the doses administered of the agents described herein may depend on various of such parameters. In the case that a reaction in a patient is insufficient with an initial dose, higher doses (or effectively higher doses achieved by a different, more localized route of administration) may be used.

According to the present invention, suitable and therapeutically effective dosages for the administration of a therapeutic agent for administration to a human subject for the treatment and/or prevention of a brain injury, in particular traumatic brain lesion or brain ischemic hypoxic lesion, including but not limited to hypoxic-ischemic encephalopathy can be determined based on experimentally determined suitable and therapeutically effective dosages for the administration of a therapeutic agent for administration to a rodent subject, particularly, a mouse, for the treatment and/or prevention of brain injury, in particular traumatic brain lesion or brain ischemic hypoxic lesion, including but not limited to hypoxic-ischemic encephalopathy.

Animal models (Examples 2 and 3) are helpful in establishing pharmacological responses, as well as assessing potential toxicities of treatment products. In some embodiments the dose to be administered to the subject is a dose as disclosed in Example 2 or in Example 3.

Preferably, the dose of the polypeptide is determined at or before the onset of treatment. In one embodiment, the dosing is adjusted for later administration(s), depending on the progression of the treated condition. In an alternative embodiment, the dosing is not adjusted for later administration(s), so that subsequent dosages correspond to the first dose.

The polypeptide is active both via nasal and systemic administration. Specifically for nasal administration the preferred dose/each dose per subject has an amount of 0.10 to 1.00 mg/day, preferably 0.06-0.12 mg/day of the polypeptide per nostrils, still preferably about 0.06 mg/day of the polypeptide per nostrils. These doses may be administered to one nostril or divided across both nostrils, according to prevailing clinical circumstances such as recipient preference, tolerable volume load and nostril access. Most preferably, these indicated dosages are specifically for administration to human.

In the following, compositions comprising the polypeptide of the present invention will be described. Such compositions are part of the present invention both as such, as well as in the specific context of use in the prevention and/or treatment of brain injury, in particular traumatic brain lesion or brain ischemic hypoxic lesion, including but not limited to hypoxic-ischemic encephalopathy according to the present invention. Thus, the present invention is directed at the medical use of such compositions.

In the compositions according to the present invention, the polypeptide of SEQ ID NO: 1 is comprised as active ingredient. Further ingredients may be comprised.

In one embodiment, the polypeptide is comprised in an aqueous medium. Said aqueous medium is for administration to the mammalian subject.

A particular aqueous composition for use according to the present invention is a liquid composition suitable for intranasal use. The formulation has a concentration 2 mg/mL, 20 mM acetate buffer, 20 mM methionine, pH 5.5. It is dissolved in 0.9% saline and may be intranasally delivered, preferably at the dose of 20 µg/kg or 40 µg/kg (4 µL total volume, 2 µL/nostril), alternating nostrils, preferably with an interval of 1-5 min, preferably 2 min between doses. Methods for making such compositions are known in the art.

In some embodiments, the polypeptide of SEQ ID NO: 1 described herein is comprised in a composition, such as a liquid composition, comprising additionally one or more carriers and/or one or more excipients. The term "carrier" as used herein refers to an organic or inorganic component, of a natural or synthetic nature, which is combined together with the active ingredient in order to enable, enhance or facilitate application of the active ingredient. The term "excipient" as used herein is intended to indicate all substances which may be present in a pharmaceutical composition of the present invention and which are not active ingredients such.

Preferably the composition according to the present invention comprises at least water as an excipient. In some embodiments, the composition according to the present invention comprises aqueous media, and more preferably the composition according to the present invention is in the form of an aqueous solution. In one embodiment, the polypeptide is comprised in an aqueous medium, and the aqueous medium is administered to the mammalian subject. The aqueous medium may be for example an aqueous solution.

Thus, the polypeptide of SEQ ID NO: 1 described herein may be present in a composition, e.g. in a pharmaceutical composition. The compositions described herein are preferably sterile and preferably contain the polypeptide of SEQ ID NO: 1 as a pharmaceutically active peptide or protein, and optionally of further agents, mentioned or not mentioned herein. The compositions may be in any state, e.g. liquid, frozen, lyophilized, etc.

The compositions described herein may comprise salts, buffer substances, preservatives, carriers, diluents and/or excipients, all of which are preferably pharmaceutically acceptable. The term "pharmaceutically acceptable" describes something non-toxic and/or which does not interact with the action of the active ingredient of the pharmaceutical composition.

Suitable buffer substances for use in the invention include 20 mM acetate buffer.

The composition (formulation) according to the present invention may be conserved, without limitation, according to one or more of the following embodiments:
- First embodiment: the above formulation can be stored frozen at -70 degrees C or at - 20 degrees C (data not shown), and thawed before administration.
- Second embodiment: the above formulation can be stored in the refrigerator, preferably in the temperature range of +2 to + 8 degrees C (data not shown).
- Third embodiment: the above formulation can be subjected to drying or freeze-drying, and then stored, e.g. at room temperature or at 2-8 degrees C (data not shown). It can be reconstituted before administration.

Suitable preservatives for use in the compositions according to the present invention include those known in the art, among which are for illustration but without limitation benzyl alcohol, benzalkonium and its salts, M-cresol, phenol, chlorobutanol, paraben and thimerosal. These and other preservatives are optionally included in the composition according to the present invention.

Thus, the present invention provides polypeptide of SEQ ID NO: 1 for therapeutic use, i.e. for the use in a method of treatment of the human or animal body by therapy. Therapy may include prevention and/or treatment of a condition. In view of the potential for therapeutic use, said polypeptide can also be referred to as a pharmaceutically active protein or peptide.

Optionally the administration according to the present invention is accompanied by administration of at least another therapeutic agent, such as agents intended to counter inflammation, oedema/swelling, raised intracranial pressure, infection, seizure, pain, psychological sequelae, etc. The other therapeutic agent may be part of the composition comprising the polypeptide according to the present invention, or alternatively may be administered to the subject separately, to the same or a different site, by the same or a different route of administration.

The carrier can be an isotonic solution, optionally buffered, such as saline solution or phosphate buffer, and can further comprise, if necessary, appropriate preservatives or other excipients suitable for the formulation of compositions for intranasal administration.

The administration can be performed for example 1 , 2, 3, 4, 5, 6, 7 or more times per day, depending on the patient's needs, and each time the dose can be, as described above, from 0.01 to 1.00 mg of CHF6467 per day.

In one embodiment, for example, the composition can be administered 2-4 times per day, for example 3 times per day, at a dose of 0.02 to 0.04 mg of CHF6467 per each administration.

For example, the composition can be administered 3 times per day in a dose of 0.02 mg CHF6467 and/or molecules with NGF-like action per administration.

The composition can be administered in any of the ways and doses described above for one or more therapeutic cycles.

Such therapeutic cycles for example can last between one day and two weeks.

By way of example, the pharmaceutical composition can be provided in the form of a number (for example any number from 10 to 20) of pre-prepared aliquots, each containing, by way of non-limiting example, 1 ml of physiological solution or any other suitable carrier, in which 0.5-2.0 mg of CHF6467 are diluted. Such aliquots can be contained in phials, or also, in an embodiment offering particular ease of use, in pre-dosed syringes suitable for connection to an MAD device. The embodiment of the aliquots is provided so as to give a possibility of administering a dose between 0.01 and 1.00 mg of CHF6467 per day. In accordance with a possible embodiment, the composition of the invention can comprise, for example, 1 ml aliquots containing 1 mg of CHF6467, this type of formulation being particularly suitable for children with a body weight around 10 kg. Alternatively the composition can comprise 1 ml aliquots of a suitable carrier with 2 mg of CHF6467, for example, for children with a body weight around 20 kg, and so on. In the knowledge of the dose advised in the present description, preparing suitable aliquots in the pharmaceutical composition by body weight range is a simple implementation for a person skilled in the art.

The possibility of administering drugs intranasally, with the objective of carrying them towards the brain, bypassing the blood-brain barrier, was already suggested more than a decade ago and is currently used for the administration of some types of sedative or anesthetic drugs, for which, by means of intranasal administration, it is possible to reduce the dose and limit them, and therefore also the possible side effects.

In accordance with a further embodiment, said one or more aliquots can be pre-calibrated for pediatric administration or for administration in adults and can be subdivided into single doses per body weight range comprising a suitable amount of CHF6467.

In an alternative embodiment, said one or more aliquots can be multi-dose aliquots, for example "daily" or "weekly" aliquots which can be subdivided into a suitable dose based on patient weight.

As already mentioned above, in accordance with one embodiment said one or more devices for administration of the composition can be devices of the MAD (mucosal atomizer device) type.

By way of non-limiting example, CHF6467may be in the form of a kit. The kit of the invention can comprise a number (for example any number from 10 to 20) of pre-prepared aliquots, each containing, by way of non-limiting example, 1 ml of physiological solution, in which 0.01-1.00 mg of CHF6467, are diluted, which, for the 10 pre-dosed syringes, gives the possibility of administering 0.01 to 1.00 mg/subject or 3 to 333 µg/kg of CHF6467. Such aliquots can be already contained in sterile syringes optionally already equipped with the MAD device for intranasal administration of the polypeptide. In accordance with a possible embodiment, the kit of the invention for example can comprise 10 syringes with 0.01 to 1 mg of CHF6467 for neonate of body weight around 3 kg. In the knowledge of the dose advised in the present description, preparing a suitable pharmaceutical kit for body weight ranges is a simple implementation for a person skilled in the art. In this way, it would be possible to safeguard the intranasal administration of a suitable amount of CHF6467 able to stimulate the intracerebral receptors for NGF (TrkA and p75) present in the majority of serotoninergic and cholinergic areas of the brain. Kits of this kind, in addition, would allow the intranasal administration of CHF6467 at home and by the parents of neonate (given the simplicity and safety of the methodology), significantly improving the quality of life of these neonates.

In addition, the composition, the kits, and the method of the invention can be used not only for patients (including patients of pediatric or neonatal age) suffering from neurological deficiencies caused by major head trauma, but also by all children affected by cerebral damage caused by hypoxic-ischemic encephalopathy brought about by perinatal damage and by patients affected generally by infantile cerebral paralysis and secondary brain injuries and cerebral ischemia of various nature (for example brain aneurysm rupture, brain arteriovenous malformations and brain sinus thrombosis).

In another preferred embodiment of the invention, the neonate is subjected to hypothermia prior to treatment with CHF6467.

In one preferred embodiment, CHF6467 is administered sequentially with hypothermia.

In one preferred embodiment, CHF6467 is administered sequentially after or before the hypothermia.

In a preferred embodiment CHF6467 is administered simultaneously with hypothermia, In one preferred embodiment of the invention, CHF6467 is administered in a therapeutically effective amount.

In another preferred embodiment, CHF6467 is administered in a sub-therapeutically effective amount.

In other words, CHF6467 is administered in an amount that would be insufficient to produce the desired therapeutic effect if administered in the absence of hypothermic conditions.

Even more preferably, the combination of CHF6467and hypothermia has a synergistic effect, i.e., the combination is synergistic.

In one particularly preferred embodiment, CHF6467 is administered prior to the hypoxic/ischemic insult itself, or else prior to diagnostic confirmation of a hypoxic/ischemic insult that is suspected to have occurred. Thus, in one preferred embodiment, CHF6467 is administered to the neonate via the mother prior to birth, for example, by administering to the mother prior to or during labour.

Preferably, CHF6467 is administered to the mother for up to about 48 or 24 hours prior to birth, more preferably up to about 12 hours, more preferably up to about 6 hours or 3 hours or 1 hour prior to birth. After birth, the neonate is then subjected to hypothermic conditions.

In another preferred embodiment, CHF6467 is administered to the neonate or newborn immediately following birth, where there are clinical grounds to suspect that a clinically impactful hypoxic/ischemic insult may have occurred, but prior to its diagnostic confirmation. This circumstance is facilitated by the speed and ease with which CHF6467 can be administered without interfering with other important clinical activities or interventions.

### EXAMPLES

### Example 1: Acute hippocampal slices undergoing oxygen-glucose deprivation (OGD)

### Methods

C57B16/J mice (30-50 days of age) were killed by decapitation and brain was rapidly removed and placed in ice-cold artificial CSF (aCSF) (0°C) oxygenated (95% O2 to 5% CO2). Parasagittal hippocampal slices (250-350 µM) were cut using vibratome and recovered for 1 h in aCSF at room temperature. A single slice was placed on a nylon mesh, completely submerged in a recording chamber and superfused with aCSF (3 mL/min, ~30°C) continuously oxygenated. Extracellular field recordings were performed using a bipolar electrode placed in Schaffer collateral fibers to elicit field excitatory postsynaptic potential (fEPSP) that was recorded with glass microelectrodes, filled with aCSF placed in the CA1 region of the stratum radiatum. Using a whole cell patch clamp recording the inventors evaluated the neuroprotective effect of CHF6467 on single neuron parameters altered by OGD. Specifically, the inventors measured spontaneous excitatory postsynaptic currents (sEPSC) and membrane potential (Vm). The inventors also assessed the time window of neuroprotection of CHF6467 during and after OGD recording excitatory postsynaptic currents (EPSC). *In vitro* oxygen/glucose deprivation (OGD) was achieved by perfusing the slices with aCSF without glucose and gassed with 95%N2 to 5%CO2. After OGD the slices were reperfused with aCSF containing glucose and O2. Hypothermia was achieved by perfusing the slices with aCSF (+4°C) containing glucose and oxygenated (95% O₂ to 5% CO₂).

### Results

The inventors studied the neuroprotective effects of CHF6467 and hypothermia alone or in combination in mouse acute hippocampal slices exposed to OGD.

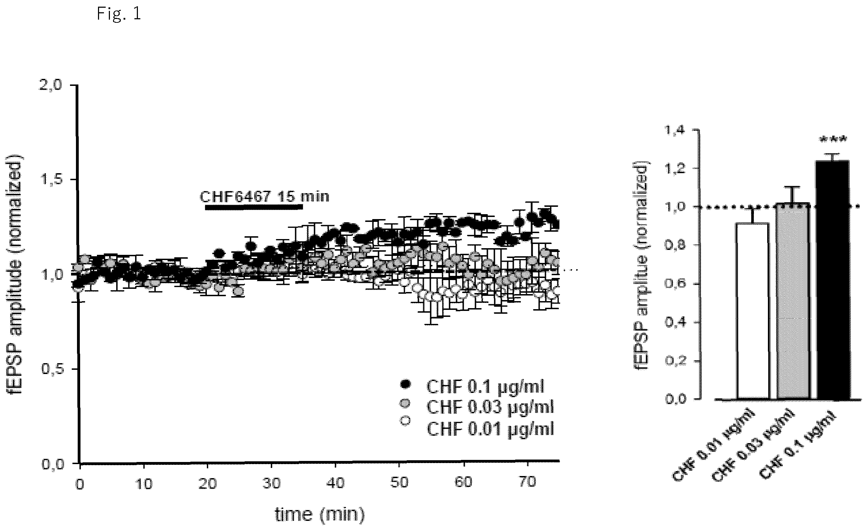

First, they investigated the effect of CHF6467 on basal neurotransmission in hippocampus. The inventors performed experiments recordings fEPSC in control condition for 20 min, then they added CHF6467 to the bath solution at different concentration (0.001-0.1 µg/mL) for 15 min. CHF6467 was able to increase the amplitude of fEPSC at 0.1 µg/mL concentration (Figure 1).

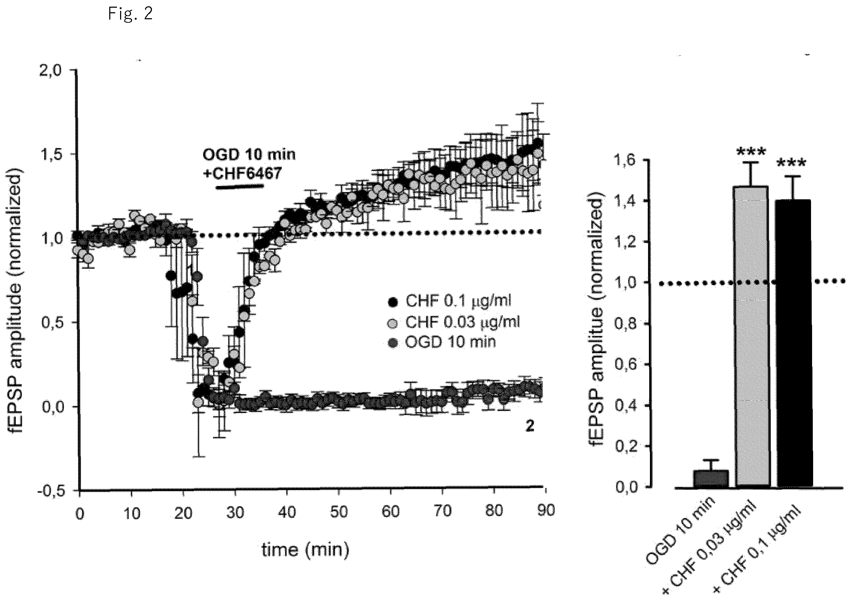

The ability of hippocampal slices to recover synaptic function on return of normal oxygenation depends on the duration of the OGD. In inventors' experimental conditions, OGD applied for 10 min induced an irreversible fEPSP depression (data not shown). Bath application of CHF6467 during OGD reverted depression of fEPSP, observed in control conditions. Slices treated with CHF6467 during OGD showed a complete recovery of fEPSP amplitude (Figure 2).

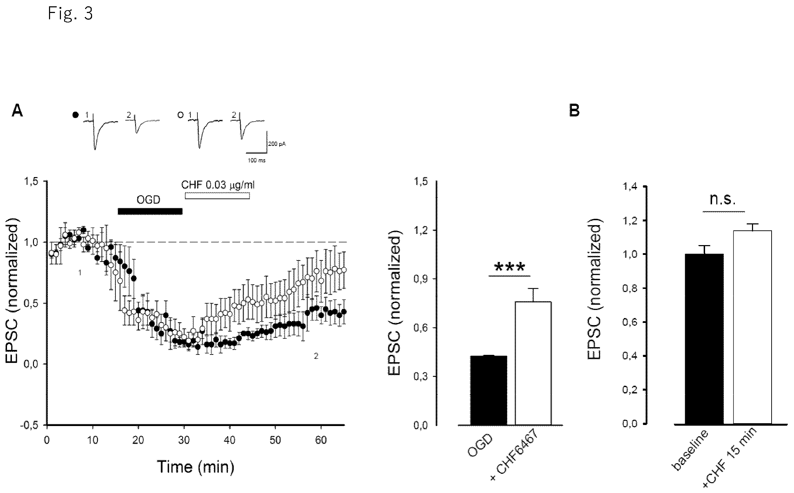

To study the therapeutic windows in inventors' in vitro model experiments in whole cell patch clamp configuration evaluating the neuroprotective effect of CHF6467 on single neurons were performed. The inventors recorded evoked excitatory postsynaptic currents (EPSC) that were irreversibly reduced after 15 min of OGD. The inventors observed a neuroprotective effect of CHF6467 when it was applied after OGD (Figure 3A), whereas no change in basal neurotransmission was observed when CHF6467 was applied under normal conditions (Figure 3B).

To investigate the possibility of a synergic effect between CHF6467 and hypothermia, which so far represents the only therapeutic approach approved for HIE, the inventors performed experiments applying CHF6467 with hypothermia after OGD. In inventors' experimental conditions, hypothermia was able to partially revert the reduction of EPSC induced by OGD (Figure 4). The co-application of CHF6467 during hypothermia was able to completely rescue the OGD reduction of EPSC (Figure 4).

The *in vitro* and *in vivo* doses of CHF6467 and the administration protocols were selected in dose-response studies to support a potential clinical application. The maximal attenuation of neuronal damage *in vitro* was observed at 0.03 µM CHF6467. Surprisingly, the neuroprotective effects of CHF6467 *in vitro* were dramatically enhanced by hypothermia at 4°C up to an almost complete reversal of neuronal electrophysiological damage.

Overall, the present electrophysiological data support the neuroprotective potential of CHF6467 against several functional alterations induced by ischemia.

In the light of these in vitro results showing that CHF6467 is able to protect from ischemia-mediated excitotoxicity (data not shown) and without being bound to any theory, CHF6467 may serve as a potential neuroprotective therapy, in combination with hypothermia, for the treatment of brain injury or traumatic brain lesion or brain ischemic hypoxic lesion, including but not limited to hypoxic-ischemic encephalopathy.

### Example 2: Rat model of neonatal hypoxic-ischemic encephalopathy

### Methods

*Animals.* Experiments and animal use procedures were in accordance with the National Institutes of Health Guide for the Care and Use of Laboratory Animals (NIH Publications No. 80-23, revised 1996). The experimental protocols were approved by the Animal Care Committee of the Department of Health Sciences, University of Florence, in compliance with the European Convention for the Protection of Vertebrate Animals used for Experimental and Other Scientific Purposes (ETS no. 123) and the European Communities Council Directive of 24 November 1986 (86/609/EEC). All efforts were made to minimize the number of animals used and their suffering.

*Procedures.* Surgical procedures were performed on postnatal day 7 Wistar rats (Harlan, MI, Italy), following the Rice-Vannucci model (Ann Neurol 1981; 9: 131-141). Briefly, rat pups were anesthetized with isoflurane gas (induction, 4%; maintenance, 1%) in oxygen:nitrous oxide (1:1). The left common carotid artery was ligated using 4-0 silk following a midline incision. After the operation, all pups were returned to their dams for recovery and lactation for 1.5 h. Then pups were placed in enclosed, vented Plexiglas chambers (W20, D20, H17) partially submerged in a water-bath and exposed by continuous flow to warmed, humidified gas 8% oxygen 92% nitrogen for 120 min. After the insult, the pups were returned to their dams for 1 h, and then placed in a chamber at normothermic (37°C) or hypothermic (32°C) temperatures for 4 h. CHF6467 was administered intranasally immediately and 24 h after hypoxia at the dose of 20 µg/kg that was shown to be well tolerated in previous experiments. All pups were then maintained with their dam and sacrificed by decapitation under isoflurane anesthesia 72 h later. For the measurement of the infarct areas and volumes, 1 mm thick coronal sections were cut with a blade, incubated in 2% 2,3,5- triphenyl-tetrazolium chloride (TTC) for 20 min at 37°C, and then fixed in 4% paraformaldehyde for 24 h. Infarct areas were measured using a computerized image analysis system (Image-Pro Plus 3.0, Silver Spring, MD, U.S.A.). The infarct volume was calculated as previously described (Ann Neurol 1981; 9: 131-141).

*CHF6467 intranasal formulation and administration.* After the end of hypoxia, rats were placed in a supine position. CHF6467 (batch no. NGF183704-TR1, concentration 2 mg/mL, 20 mM acetate buffer, 20 mM methionine, pH 5.5) was dissolved in 0.9% saline and intranasally delivered at the dose of 20 µg/kg (4 µL total volume, 2 µL/nostril), alternating nostrils with an interval of 2 min between doses.

*Statistical analysis.* Data are presented as means ± SEM of n experiments. Statistical significance of differences between propidium iodide fluorescence intensities or TTC staining was analyzed using two-way ANOVA followed by the Holm-Sidak test for multiple comparisons. All statistical calculations were performed using SigmaPlot (version 11.0). A probability value (P) of < 0.05 was considered significant.

### Results

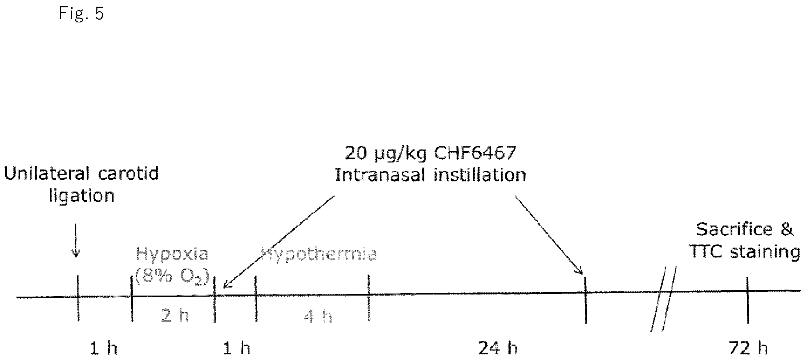

The inventors studied the neuroprotective effects of CHF6467 alone or in combination with hypothermia in the neonate rat model of neonatal hypoxic-ischemic encephalopathy (HIE) (Rice-Vannucci model; Ann Neurol 1981; 9: 131-141). The experimental protocol is shown in the diagram (Figure 5). In this model, left carotid artery occlusion followed by exposure to hypoxia (8% O2 and 92% nitrogen for 2 h) results in an ~80% occurence of ipsilateral cerebral infarction in vehicle-treated rat pups as described previously (Landucci et al., Neurosci Lett . 2018; 668:103-107).

Observational findings on the fresh hemisphere ipsilateral to the carotid artery occlusion included pallor, atrophy, and tissue loss in the striatum, hippocampus, cortex and thalamus (data not shown). When brain slices were stained with 2,3,5-triphenyltetrazolium chloride (TTC), the inventors observed that HIE produced a dramatic brain infarct (Figure 6) that was 70.4 ± 7.8 mm³ in volume.

After the end of hypoxia, rats were placed in a supine position. CHF6467 was dissolved in 0.9% saline and intranasally delivered at the dose of 20 µg/kg or 40 µg/kg (4 µL total volume, 2 µL/nostril), alternating nostrils with an interval of 2 min between doses. Administration of 20 and 40 µg/kg (equivalent to about 60 to 120 µg for a human neonate of 3 kg body weight) CHF6467 intranasally immediately and 24 h after hypoxia significantly reduced the extent of the infarct compared to vehicle.

Hypothermia (32°C for 4 h beginning 1 h after the end of hypoxia) significantly reduced the extension to vehicle-treated animals in normothermia (Figure 6). The combination of CHF6467 20 µg/kg with hypothermia elicited a dramatic reduction of the infarct volume that was significantly greater than that produced by hypothermia alone (Figure 6). Interestingly and surprisingly, the combination of hypothermia and CHF6467 neuroprotection superseded the sum of the neuroprotective effects of the single agents.

The mean percent reductions of the mean Log-transformed brain infarcted volume of the vehicle + normothermia group after the different treatments were:
- Normothermia + CHF6467 20 µg/kg: -13.6%
- Normothermia + CHF6467 40 µg/kg: -24.3%
- Hypothermia + Vehicle: -13.4%
- Hypothermia + CHF6467 20 µg/kg: -47.9%

Animals were monitored once daily to examine clinical signs and twice daily for mortality and morbidity.

### Analysis of interaction effect

In the following statistical analyses were performed using SAS version 9.4 (SAS Institute Inc., Cary, NC, USA). The statistical analysis of interaction effect between CHF6467 and hypothermia was based on log10-transformed data, using an ANOVA model assuming a separate variance for each treatment group. The fixed effects of hypothermia (yes/no), CHF6467 (yes/no) and their interaction were included in the model. Data from the group treated with normothermia + CHF6467 40 µg/kg were excluded from the analysis because they were not contributing to the assessment of the interaction effect (as this study did not include a group hypothermia + CHF6467 40 µg/kg).

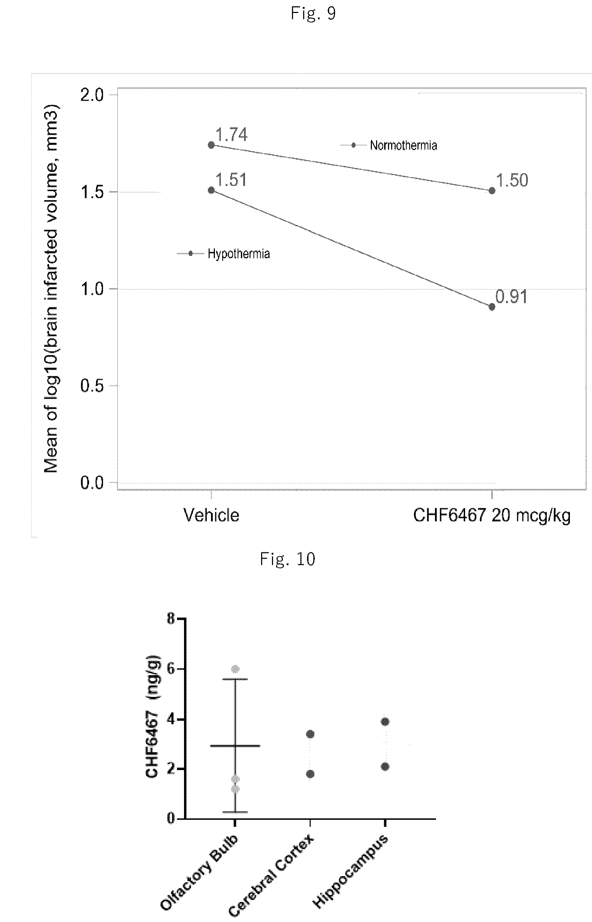

The interaction between CHF6467 20 µg/kg and hypothermia was statistically significant (p = 0.006). This interaction is apparent in Figure 9, where the mean log10-transformed brain infarcted volume is presented by treatment group. In case of additive effects (no interaction) of CHF6467 20 µg/kg and hypothermia, parallel lines would be expected. The absence of parallelism suggested an interaction between the effects of CHF6467 20 µg/kg and hypothermia. The neuroprotective effect of the combination of CHF6467 20 µg/kg and hypothermia was larger than the sum of the individual effects of CHF6467 and hypothermia.

Table 1 reports the effect of CHF6467 alone or combined with hypothermia on body weight. No statistically significant differences in body weight gain and no adverse clinical signs were observed.

**Table 1: Effect of CHF6467 alone or combined with hypothermia on body weight**

| | **0 h** | **24 h** | **48 h** | **72 h** |
|---|---|---|---|---|
| **SHAM** | 17.17 ± 1.20 | 19.44 ± 1.27 | 21.44 ± 1.28 | 23.02 ± 1.69 |
| **HIE** | 16.84 ± 0.38 | 17.44 ± 0.46 | 19.72 ± 0.58 | 21.87 ± 0.88 |
| **HIE+ CHF6467 20µg/Kg i.n.** | 15.33 ± 0.66 | 16.28 ± 0.58 | 18.48 ± 0.62 | 21.35 ± 1.15 |
| **HIE+ CHF6467 40µg/Kg i.n.** | 17.22 ±0.38 | 18.14 ± 0.53 | 20.67 ± 0.53 | 23.00 ± 0.45 |
| **HIE+ IPO** | 15.68 ± 0.46 | 17.12 ± 0.62 | 19.03 ± 0.77 | 21.49 ± 1.12 |
| **HIE+ IPO+ CHF6467 20µg/Kg** | 15.31 ± 0.44 | 16.90 ± 0.56 | 19.17 ± 0.73 | 21.49 ± 1.00 |

Then, CHF6467 may be used as a neuroprotective agent in neonates with HIE in combination with hypothermia.

### Example 3: Effect of CHF6467 on neuroinflammatory markers expression in a juvenile rat model of hypoxic-ischemic brain injury

### Methods

*Experimental animal procedures.* All the procedures and conditions involving experimental animals were reviewed and approved by the local ethics committee and authorized by the Italian Ministry of Health (authorization number 671/2019-PR).

The experimental procedure were conducted as described previously (Landucci et al., 2018 Neurosci. Lett. 668). In brief, postnatal day 7 Wistar rat pups were anesthetized with isoflurane gas (induction, 4-5%; maintenance, 1-2%). The left common carotid artery was ligated using 4-0 silk following a midline incision. After the operation, all pups were returned to their dams for recovery and lactation for 1 h. Then pups were placed in enclosed, vented Plexiglas chambers (W20, D20, H17) partially submerged in a water-bath and exposed by continuous flow to warmed, humidified gas 8% oxygen 92% nitrogen for 120 min. After the insult, the pups were returned to their dams for 1 h, and then placed in a chamber at normothermic (37°C) or hypothermic (32°C) temperatures for 4 h. CHF6467 was administered intranasally immediately after the end of hypoxia at the dose of 20 µg/kg. All pups were then maintained with their dam and sacrificed by decapitation 24 h later.

*CHF6467 intranasal formulation and administration.* After the end of hypoxia, rats were placed in a supine position. CHF6467 (batch no. NGF183704-TR1, concentration 2 mg/mL, 20 mM acetate buffer, 20 mM methionine, pH 5.5) was dissolved in 0.9% saline and intranasally delivered at the dose of 20 µg/kg or 40 µg/kg (4 µL total volume, 2 µL/nostril), alternating nostrils with an interval of 2 min between doses.

*RNA extraction from hippocampal and cortical brain areas.* Phenol/guanidine-based lysis of samples: 800u1 of QIAzol Lysis Reagent /30mg tissue for both hippocampus and cortex were used. Tissue samples were homogenized combining beads and Tissue Lyser (Qiagen) device. After addition of chloroform, the homogenate was separated into aqueous and organic phases by centrifugation. The upper, aqueous phase was extracted by QiaCube Connect automable system (Qiagen). Protocol including DNase step was chosen in order to remove gDNA. RNA samples were eluted in RNase free water and stored at -80°C until Reverse transcription step.

Concentration of RNA samples was determined at Nanodrop (ThermoFisher) analyzer and adjusted in order to retrotranscribe 1000ng of RNA in one RT reaction for each sample. An additional ezDNase^{™} Enzyme step was performed before RT reaction in order to remove gDNA. Thermal cycling conditions suggested by manufacturer were followed to allow RT reaction to.

*Panel of neurinflammatory markers.* cDNA samples were tested on custom designed array plates (Taqman gene array, Thermofisher) including primers and probe for target and housekeeping genes.

The following genes (Thermofisher catalog number in brakets) were targeted during the study: Housekeeping genes: 18s rRNA (Hs99999901_s1), B2m (Rn00560865_m1), Tbp (Rn01455648_m1); Target genes: Ccl2 (Rn00580555_m1), Ccl22 (Rn01536591_m1), Ccl12 (Rn01464638_m1), Tnf (Rn00562055_m1), Cxcl2 (Rn00586403_m1), Ccl7 (Rn01467286_m1), Ccr5 (Rn02132969_s1), Ccl20 (Rn00570287_m1), Il6 (Rn01410330_m1), Cxcl10 (Rn01413889_g1), Ccl6 (Rn01456400_m1).

TaqMan^{®} Fast Advanced Master Mix (Applied Biosystems) and diluted cDNA were added to plates; thermal cycling conditions suggested by manufacturer were followed to carry out PCR reaction. Results were calculated using the 2-ΔΔCt method based on housekeeping gene amplification.

Statistical analysis was performed by Graph Prism software; one-way ordinary ANOVA test followed by Dunnett or Tuckey ad-hoc comparisons were applied.

### Results

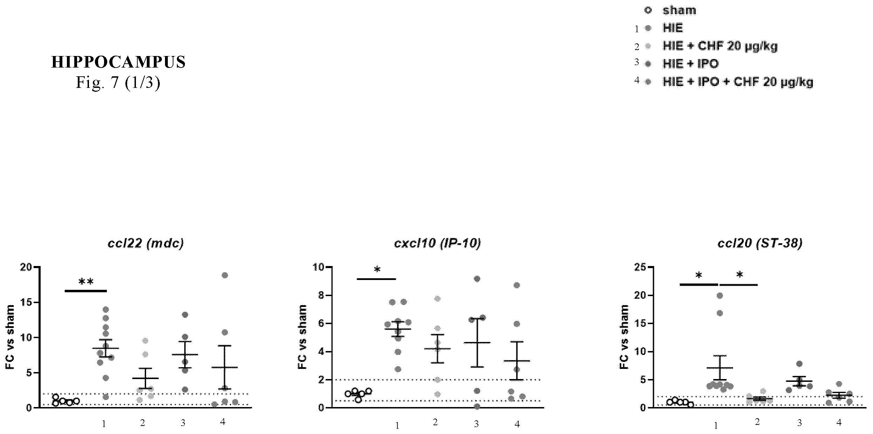

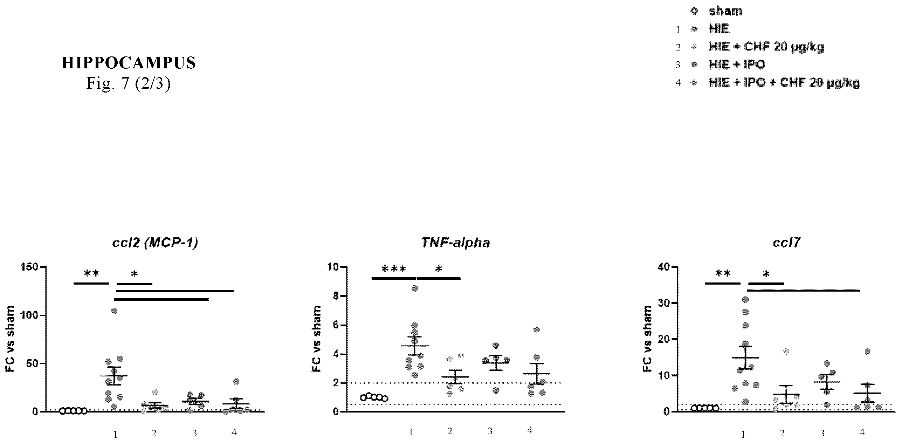

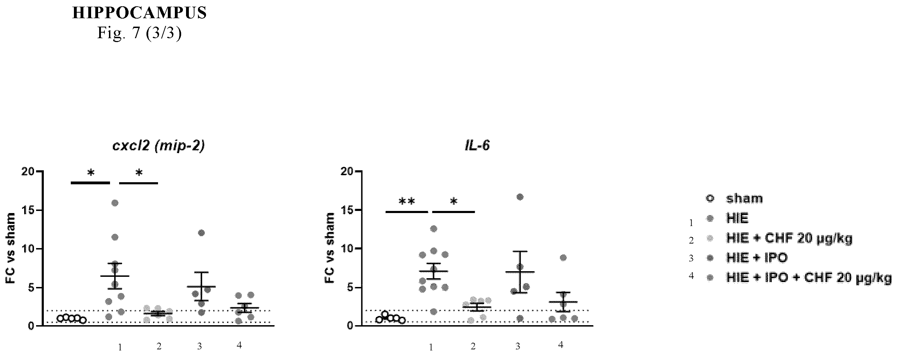

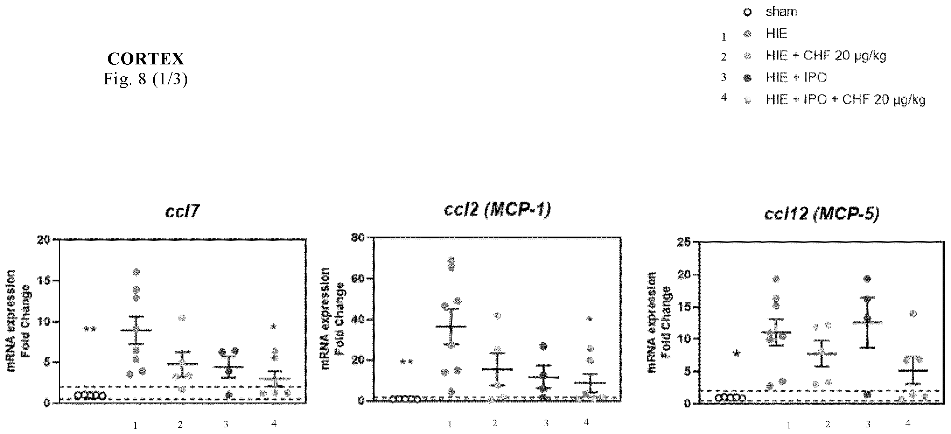

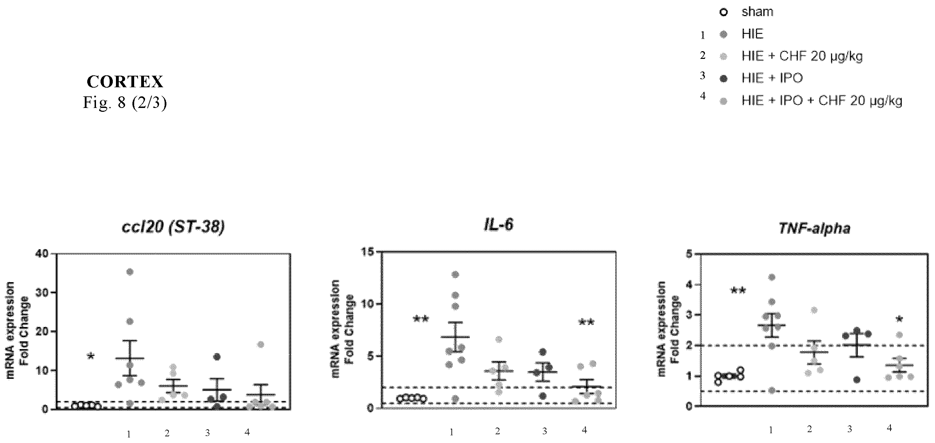

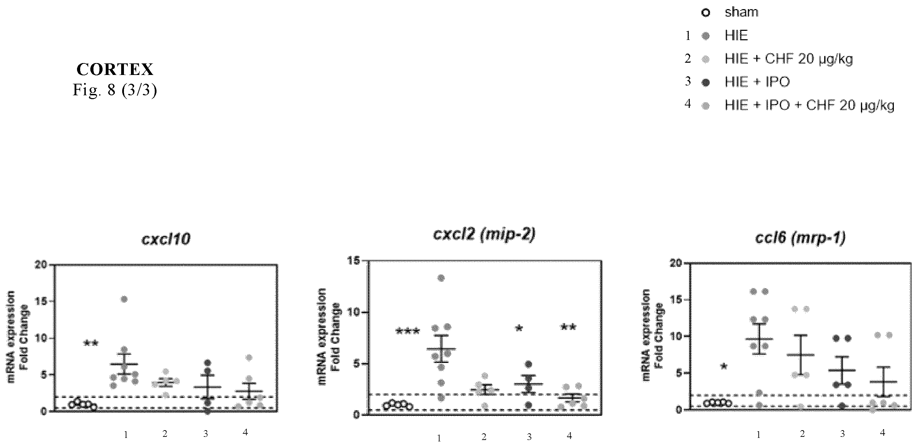

A marked increase of mRNA levels of a panel of cytokines and chemokines in the hippocampus and the frontoparietal cortex was measured in rats that underwent hypoxic-ischemic insult in comparison with sham-operated rats (Fig. 7 and 8). CHF6467 (20 ug/kg, intranasal) significantly counteracted the upregulation of several neuroinflammatory markers (ccl2, cxcl2, TNF-alpha, IL-6, ccl20) in the hippocampal region of rats subjected to the hypoxic-ischemic insult while hypothermia per se was not as effective as CHF6467 although counteracted significantly ccl2 upregulation (Fig 7).

In the cortex, CHF6467 (20 ug/kg) per se counteracted some neuroinflammatory markers upregulation (but only cxcl2 reached a statistical significance) induced by the hypoxic-ischemic insult and such effect was, for some neuroinflammatory markers (ccl7, ccl2, IL-6, TNF-alpha, cxcl2), augmented by the combination with hypothermia (Fig. 8).

Moreover, the combination of CHF6467 and hypothermia augmented the effect of the single treatments against ccl2, ccl7, TNF-alpha and IL-6. Then, CHF6467 can exert major modulatory effects versus the immunoinflammatory response evoked by hypoxiaischemia in the immature rodent brain, and such effect is non-redundant when combined with hypothermia.

### Example 4: Quantification of CHF6467 in rat brain

### Methods

### Peptides selection and synthesis of heavy labeled standards

Four peptides were selected showing different specificities for CHF6467 and/or mature rat NGF (Table 2) and their corresponding AQUA (Absolute QUAntification) homologues were synthetized by Thermo Scientific.

**Table 2. NGF peptides selected for mass spectrometry detection.**

| **Specificity** | **Sequence** | **Start** | **End** | **MWₘₒₙₒ** |
|---|---|---|---|---|
| **CHF6467, Human** | SSSHPIFHR | 1 | 9 | 1066.531 |
| SEQ ID No. 2 | *SSSHPIFH**R** (L-Arginine-¹³C₆,¹⁵N₄ , +10Da) | | | 1076.539 |
| **CHF6467** | QAAWEFIR | 96 | 103 | 1019.519 |
| SEQ ID No. 3 | *QAAWEFI**R** (L-Arginine-^{13C}6,¹⁵N₄ , +10Da) | | | 1029.527 |
| **Unspecific** | QYFFETK | 51 | 57 | 961.455 |
| SEQ ID No. 4 | *QYFFET**K** (L-Lysine-¹³C₆,¹⁵N₂, +8Da) | | | 969.469 |
| **Rat** | ALTTDDK | 89 | 95 | 762.376 |
| SEQ ID No. 5 | * ALTTDD**K** (L-Lysine-¹³C₆,¹⁵N₂, +8Da) | | | 770.390 |

| | | | | |
|---|---|---|---|---|
| * Sequences isotopically enriched for AQUA assay: heavy isotopes (¹³C₆,¹⁵N₄) for Arginine and (¹³C₆, ¹⁵N₂) for Lysine were added to the last amino acid as indicated in bold font. | | | | |

### Experimental animal procedures

All the procedures and conditions involving experimental animals were reviewed and approved by the local ethics committee and authorized by the Italian Ministry of Health (authorization number 671/2019-PR). As described previously (Landucci et al., 2018, Neurosci. Lett. 668. doi:10.1016/j.neulet.2018.01.023.), postnatal day 7 Wistar rat pups were anesthetized with isoflurane gas (induction, 4-5%; maintenance, 1-2%). The left common carotid artery was ligated using 4-0 silk following a midline incision. After the operation, all pups were returned to their dams for recovery and lactation for 1 h. Then pups were placed in enclosed, vented Plexiglas chambers (W20, D20, H17) partially submerged in a water-bath and exposed by continuous flow to warmed, humidified gas 8% oxygen 92% nitrogen for 120 min. After the insult, the pups were returned to their dams for 1 h, and then placed in a chamber at normothermic (37°C) or hypothermic (32°C) temperatures for 4 h. CHF6467 was administered intranasally immediately after the end of hypoxia at the doses of 40 µg/kg. In addition, a group of naive animals that did not undergo the hypoxic-ischemic insult, was administered intranasally with 40 µg/kg. All pups were then maintained with their dam and sacrificed by decapitation 2 h later.

### CHF6467 intranasal formulation and administration

After the end of hypoxia, rats were placed in a supine position. CHF6467 (concentration 2 mg/mL, 20 mM acetate buffer, 20 mM methionine, pH 5.5) was dissolved in 0.9% saline and intranasally delivered at the dose of 40 µg/kg (4 µL total volume, 2 µL/nostril), alternating nostrils with an interval of 2 min between doses.

### Protein extraction from hippocampal and cortical brain areas

Thawed brain samples are kept in ice and Extraction Buffer is added in the ratio 10:1 (v/w) (50 mM ABC + 2% SDS + Protease Inhibitor Cocktail dil.500X). Tissue homogenization is performed by TissueLyser II bead milling, 2 minutes at 30/s. Then the samples are kept for 5 minutes at 70 °C and allowed to cool at room temperature before 15-minute treatment with 25 U/mL Benzonase. Protein content is quantified by BCA assay.

### Protein digestion

Brain homogenates are reduced (5 mM DTT, 5 minutes at 95 °C) and alkylated (15 mM IAA, 45 minutes at 24 °C in the dark). Detergent is removed from the sample buffer through Pierce spin columns. Then 20% ACN is added to the digestion buffer before adding Trypsin in 1:20 (w/w) ratio, 5 hours at 37 °C followed by a second addition of Trypsin in 1:20 (w/w) ratio and overnight incubation at 37 °C. Before quenching the reaction with 0.1% TFA, all the samples are doped with 1.5 ng/g AQUA peptides.

### Sample fractionation

Digested samples are fractionated by size exclusion chromatography through ÄKTA Pure 25M system equipped with a Superdex 30 Increase 3.2/300 column and a F9-R collector, running a linear gradient of 30% ACN, 0.1% TFA.

### CHF6467 detection and quantification

Selected SEC fractions are collected, SpeedVac dried and resolubilized in 3% ACN, 0.1% TFA before nanoLC-MS analysis through an UltiMate^{™} 3000 RSLCnano System coupled to an Orbitrap Fusion^{™} Lumos^{™} Tribrid^{™} Mass Spectrometer. NanoLC is operated in preconcentration mode and separation is performed through an EASY-Spray PepMap RSLC C18 column, 50 cm x 75 µm. Mobile phase gradient is 5-40 %B in 100 minutes where B is 80% ACN and 0.1% FA. Mass spectrometry analysis consist of full scan and timed PRM acquisition to detect the CHF6467 peptides of interest and their corresponding heavy labeled peptides. Tentative calibration curves were built to quantify the levels of CHF6467 signal in brain matrix. A 8-point concentration range between 0.09 and 12 ng/g was initially tested for the analysis of tissues from control animals, then a 6-point concentration range between 0.06 and 13.5 ng/g was tested prior to the analysis of HIE animals.

### Results

The sequence analysis of natural mature human and rat NGF isoforms as well as the CHF6467 variant led the inventors to identify long homology regions but also some unique tryptic peptides (Table 2). Inventors selected two peptides that, considering the animal model in this study, are proteotypic of CHF6467 variant: SSSHPIFHR (m/z 534.265) and QAAWEFIR (m/z 510.759). They also monitored the contribution of a peptide potentially reporting on both the exogenous and endogenous isoforms: QYFFETK (m/z 481.727, SEQ ID No. 4). Lastly, they identified a rat-selective peptide, ALTTDDK (m/z 382.188, SEQ ID No. 5), to specifically report on a potential endogenous response.

Inventors preliminary tested the distribution of CHF6467 after intranasal administration in control juvenile rats. This proof of principle was performed on a small number of samples: three olfactory bulbs, two hippocampi and two cerebral cortexes. CHF6467 was detected in all the samples, providing evidence of its ability to penetrate the brain areas of interest. Quantification estimates, performed based on SSSHPIFHR (SEQ ID No. 2) peptide signals (Figure 10), showed that, on average, few hundreds of picograms can be found in the cortical areas and in the hippocampi.

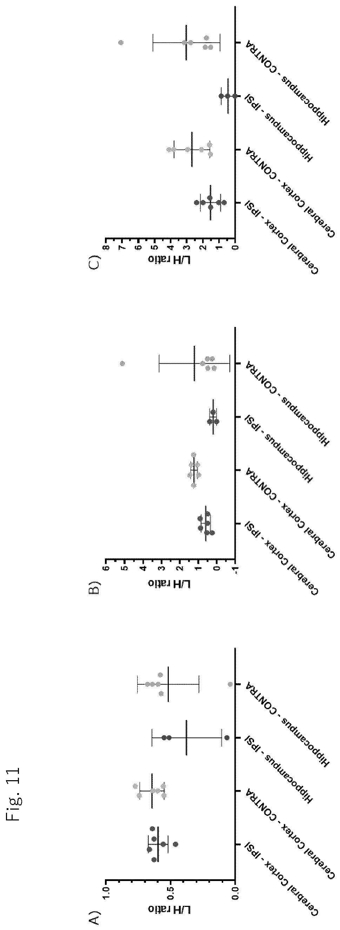

Inventors follow up this study with the investigation of the extent of brain penetration of CHF6467 intranasally administered to juvenile rats subjected to hypoxic-ischemic insult (Landucci et al., 2018 Neurosci. Lett. 668. doi:10.1016/j.neulet.2018.01.023.). They analysed cerebral cortexes and hippocampi from six animals, comparing ipsi-lateral and contra-lateral tissues. Except for three ipsi-lateral hippocampi that were lost during sample preparation due to a crack in the tube, CHF6467 has been detected in all the analysed samples with a fair reproducibility between sample replicates within the same area (Figure 11). Based on all the three peptides selected for quantitation, the detected CHF6467 amounts appeared to be out of the quantitation range tested (0.06 and 13.5 ng/g), suggesting levels lower than 60 pg/g within this group of animals subjected to hypoxic-ischemic insult.

This study demonstrates that CHF6467 can indeed enter the brain after intranasal administration reaching the hippocampus and cerebral cortex, where it can exert its neuroprotective and anti-neuroinflammatory effect against the hypoxic-ischemic insult in the immature rodent brain.

Mass spectrometry is a valuable tool to specifically detect and quantify proteins in complex matrices. Overall, the data show that intranasal administration of CHF6467 results in detectable levels of the protein in relevant brain areas, in particular hippocampus and cortex.

## Claims

1. A polypeptide having at least 50% of the activity of a polypeptide of SEQ ID NO:1 for use, in combination with hypothermia, in the treatment and/or prevention of traumatic brain lesion or of brain ischemic hypoxic lesion in a human subject.

2. The polypeptide for use according to claim 1, wherein said polypeptide has at least 60%, 70%, 75%, 80%, 85%, 90%, 95%, 97%, 98%, or 99% of the activity of the polypeptide of SEQ ID NO:1 or is the polypeptide of SEQ ID NO:1.

3. The polypeptide for use according to claim 1 or 2, for use in the treatment and/or prevention of hypoxic-ischemic encephalopathy, preferably including primary or secondary damage.

4. The polypeptide for use according to any one of claims from 1 to 3, wherein the subject is a neonatal or newborn subject or a premature newborn subject, preferably the neonatal or newborn subject is affected by moderate to severe hypoxic-ischemic encephalopathy.

5. The polypeptide for use according to any one of claims from 1 to 4, wherein the polypeptide is administered intranasally.

6. The polypeptide for use according to any one of claims from 1 to 5, wherein the polypeptide is administered repeatedly, preferably at least three times per day, or in a single administration.

7. The polypeptide for use according to claim 6, wherein the repeated administration lasts for a period of 1 to 30 days, preferably 2 to 14 days.

8. The polypeptide for use according to any one of claims from 1 to 7, wherein the polypeptide is administered at a dose of 0.01 to 1.00 mg/day, preferably at a dose 0.06 to 0.12 mg/day of the polypeptide.

9. The polypeptide for use according to any one of claims from 1 to 8, wherein when the polypeptide is administered repeatedly, each dose is of 0.01 to 1.00 mg/day, preferably each dose is of 0.06 to 0.12 mg/day.

10. The polypeptide for use according to any one of claims from 1 to 9, wherein the polypeptide is administered within about 1 hour to 24 hours after the brain lesion, wherein about designates +/- 10 % around the numerical value or range claimed.

11. The polypeptide for use according to any one of claims from 1 to 10, wherein said polypeptide is comprised in a composition comprising an acetate buffer and/or methionine.

12. The polypeptide for use according to any one of claims from 1 to 11, wherein the polypeptide is administered simultaneously with hypothermia and/or no more than 24 hours after hypothermia.

13. The combination for use according to any one of claims from 1 to 12, wherein the hypothermia is to be maintained for a period of from about 1 to about 72 hours after the hypoxic-ischemic (HI) insult, wherein about designates +/- 10 % around the numerical value or range claimed.

14. The combination for use according to any preceding claim wherein the temperature of the mammal is to be maintained at a temperature of from about 32°C to about 36°C, wherein about designates +/- 10 % around the numerical value or range claimed.

15. The polypeptide for use according to claim 4, wherein the subject is affected by neurological deficiencies caused by major head trauma; cerebral damage caused by hypoxic-ischemic encephalopathy brought about by perinatal damage; infantile cerebral paralysis; secondary brain injuries; cerebral ischemia.
